(19) Europäisches Patentamt
European Patent Office
Office européen des brevets

(11) Publication number: 0 309 077 A2

# (12) EUROPEAN PATENT APPLICATION

(21) Application number: 88306578.1

(51) Int. Cl.⁴ A61K 31/40

(22) Date of filing: 19.07.88

(30) Priority: 21.09.87 CS 6793/87

(43) Date of publication of application: 29.03.89 Bulletin 89/13

(84) Designated Contracting States: BE CH DE FR GB IT LI NL SE

(71) Applicant: SLOVENSKA AKADEMIA VIED
Obráncov mieru 49
Bratislava(CS)

(72) Inventor: Raskova, Helena, Prof.MUdr DrSc
No. 2 v Ondrejové
Praha 4(CS)
Inventor: Bauer, Viktor, MUDr DrSc
No 17 Bratislavska
Samorin(CS)
Inventor: Urbanova, Zdenka, MVDr CSc
No 456 Jaselska
Kutna Hora(CS)
Inventor: Benes, Ludek, Doc.RNDr DrSc
No 39 Brnianska
Bratislava(CS)
Inventor: Stolc, Svorad, MUDr CSc
No 4L.Dérera
Bratislava(CS)
Inventor: Dittertova, Valéria, RNDr PhMr
No 11 Kupeckého
Bratislava(CS)
Inventor: Babulova, Anna, RNDr PhMr CSc
No 9 Mostna
Bratislava(CS)

(74) Representative: Griffin, Kenneth David et al
Saunders & Dolleymore 9, Rickmansworth Road
Watford Hertfordshire WD1 7HE(GB)

## (54) Antidiarrhoeal therapeutic agent.

(57) A medicament for the treatment of diarrhoeal diseases, especially of nonspecific charater, which is designed for oral administration and contains, as active component, (±)-trans-2(1-pyrrolidinyl)cyclohexylester of 3-(n)-pentyloxycarbanilic acid, or a salts of this base with pharmaceutically acceptable acids, and, optionally a physiologically harmless vehicle.

EP 0 309 077 A2

# ANTIDIARRHOEAL THERAPEUTIC AGENT

The invention relates to a drug for the treatment of nonspecific diarrhoea caused, among others, by enteropathogens and enterotoxins. Apart from this, the active substance according to the invention has other effects such as particularly anti-ulcerative, spasmolytic and locally anaesthetic ones.

Diarrhoea and diarrhoeal diseases are one of the most frequent causes of illness and mortality especially in less developed countries, where the yearly mortality is estimated at about 5 million persons. Diarrhoeal diseases are particularly dangerous in new-born babies and very young children (S Hughes: Drugs 26, 80-90 (1983)).

In mechanized or automated large-capacity farms, diarrhoea and infections of the respiratory tract are frequent, especially in young livestock, and a high mortality or delays in growth have a considerable adverse ecnomical effect. Diarrhoeal diseases in humans and animals are caused by a number of aetiological factors, especially of microbial and viral character. Among microbes the main culprits are gram-negative bateria, Escherichia coli, Vibrio cholerae etc.

To cause diarrhoea by microorganisms, two factors have to be present:

(a) Adherence factors in the form of specific fimbria (pili) which enable the microorganisms to adhere to epithelial cells of small intestine mucuous membrane and to reproduce. When orally received microorganisms get into the small intestine without being affected by gastric acid, they settle, after reproduction, in the colon.

(b) Some gram-negative bacteria produce toxins which affect epithelial cells and increase the activity of adenyl cyclase and cyclic adenosine-5′-monophosphate (cAMP). A higher level of cAMP leads to a predominance of liquid flow from the tissues into the small intenstine lumen. Thus the loss of liquids and ions results in dehydration and acidosis which may even endanger life.

These conditions can be coped with only to a small extent, owing to difficulties in ascertaining bacterial prophylaxis and mastering the hygienic situation or an adequate rehydration therapy in terrain practice.

For fighting diarrhoeal diseases rehydration therapy is applied using preparations composed of various salts (potassium chloride, sodium chloride, sodium hydrogen carbonate) and glucose whereby, admittedly, a quick compensation for the loss of water and ions occurs and acidosis is alleviated but the occurrence of diarrhoeal diseases is not influenced.

Also used are anticholinergic substances together with spasmolytics, such as Reasec[R] (Janssen), which contain diphenoxylate and atropine. In both human and veterinary medicine, some chemotherapeutic agents having antibacterial effect, such as sulphonamides or antibiotics, are used, which suppress certain infections.

Pharmacological intervention by means of medicaments is also aimed at the sphere of regulation depending on receptors, especially those localised on the basolateral membrane, further by means of a intracellular mechanism of the intervention by the so-called secondary messenger, and by influencing the transport mechanism, especially boundary membranes. The modulation of receptor-dependent regulation mechanisms can be influenced, to some extent, by medicaments of the type alpha$_2$ adrenergic agonists such as clonidine (Catapresan[R]) E B Chang et al: Gastroenterology 91,564-9 (1986), somatostatin or encephalin, and morphine analogs. For influencing the transport of ions through the membrane it is also possible to use apha$_2$ adrenergic agonsits (E B Chang et al: Am.J.Physiol. 1982,242). Reference has also been made to the use of lidamidine, which has a damping effect on intestinal peristalsis (M D Dharmsathphorn: Gastroenterology 91, 769-775 (1986)).

A disadvantage of antidiarrhoeal drugs, namely those referred to in literature rather than those used in practice, consists in their strong side effects, such as antihypertensive effects (clonidine), growth factors (somatostatin), drug dependence and also uncompleted preclinical research (enkephalin derivatives). The use of large doses of antiboitics and their long-termed administration has not proved to be optimum in localities with epidemic diarrhoea. In cases where the diarrhoea inducing agent is cholera toxin, no effective protection exists except for an inoculum which is not sufficiently effective either, gives a short-termed protection only (3 months) and has poor effectiveness (30 - 40%).

Some disadvantages and drawbacks of hitherto applied therapy are eliminated by the antidiarrhoeal agent according to the present invention whose active component is the base (±)-trans-2(1-pyrrolindinyl)-cyclohexylester of 3-(n)-pentyl-oxycarbanilic acid of the formula

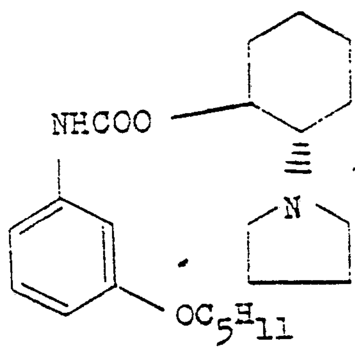

this base or its salts with pharmaceutically acceptable acids such as, preferably, hydrochloride, adipate, fumarate, optionally with a physiologically harmless vehicle in a form suitable for oral administration.

Preparation of the active substance is known and a method of its production is protected by Czechoslovak patents Nos. 125,666 and 126,102. At present, the drug containing the active substance is known and protected as a therapeutic agent with antiulcerative, spasmolytic and locally anaesthetic effects (Csechoslovak Autorship Certificate No. 237,705), which has now been found to have suprisingly antidiarrhoeal effects.

It exhibits well-known locally anaesthetic effects (L Benes et al: Arzneim. Forsch. 19,1902 (1969)), locally anaesthetic and spasmolytic effects (P Svec et al: Farm. Obzor 45,355 (1976)); the character of this effect, its initiation, development, duration and dying out as well as maintaining of its locally anaesthetic activity even in conditions with lowered pH value at the acid side have been reported (S Stolc et al: Brat.Lek.Listy 70, 297 (1978)); antiulcerative effects with a marked gastric cytoprotective effect (V Nosalova et al: New Pharmacology of Ulcer Disease, ed M Fisher, Prager Publ. London 1986, p 113); and further a significant reduction of adenyl cyclase activity (Hynie et al: Cs.Fysiol. 35, 338-339 (1986)), which is probably one of the possible mechanisms of antidiarrhoeal effects of the substance according to the above formula. A low acute toxicity, subchronic and chronic toxicity together with the newly discovered effects constitute excellent conditions for the treatment of diarrhoeal diseases. The elimination of a high dehydration of the organism resulting, among others, from the diarrhoeal disease proper, reduces the mortality in these groups of effected people and animals.

The antidiarrhoeal medicament according to the above formula, if administered in daily doses of 10 and 20 mg for one to three days after birth to calves having average weight of 40 kg, resulted in a significant reduction of diarrhoea in the effected animals, depending upon the dose applied. In a control group (n = 66) diarrhoea has occurred in about 80% of the animals. The medicament according to the invention, when administered for 2 to 3 days in a daily dose of 10 mg, has reduced the occurrence of diarrhoea by 40-50%, and, when administered in a daily dose of 20 mg for 3 days below 40% ($P< 0.01$). In the control group of calves to which the medicament according to the above formula has not been administered, a much higher morality was observed. Thus, for instance, a control group of 66 calves to which no medicament was administered, when compared with the same number of calves treated with said medicament in the form of capsules containing 10 and 20 mg thereof, had a statistically considerably highert mortality.

Similarly, the effect of the medicament according to the above formula was proved experimentally with mice in which diarrhoea was caused by castor oil. The active substance in a 20 mg dose per one kg, if administered 30 minutes before the administration of castor oil, has extended the latent period up the beginning of diarrhoea from 91.2±4.4 minutes to 127.3±6.3 minutes. It was also found that the active substance does not significantly influence the passage of bowel contents (carbo adsorbens) either with mice, or brown rats (Mus decumanus) unlike atropine as control substance (TAB. 1)

TABLE 1

| Influence of pharmaceutic substances on the intestinal passage of carbo adsorbens (100% is the whole intestine length) | | | |
|---|---|---|---|
| | mice | | brown rats |
| | small intestine | the whole intestine | small intestine |
| control | 91.7±2.2 | 75.0±1.9 | 58.7±2.5 |
| substance acc. to the formula 20 mg/kg | 96.6±0.95 | 80.3±1.2 | 60.1±1.5 |
| atropine 5 mg/kg | | 51.4±2.6 | 46.3±2.5 |

When the active substance according to the formula was affected in a dose of 10 mg/kg, the retention of the stomach content did change, while after a higher dose (20 mg/kg) it has increased similarly to the case of atropine administration.

These results suggest that the active substance according to the present invention influences the secretion processes rather than the intestine content passage.

The following TABLE 2 gives the values of acute toxicity of the substance according to the above formula, expressed as $LD_{50}$ in mg/kg with various animal species (mice, brown rat, rabbit, guinea pig):

TABLE 2

| Mean lethal doses of the substance according to the invention | | |
|---|---|---|
| mode of administration | mice | |
| | male | female |
| i.v. | 10.3 | 10.3 |
| i.p. | 56.0 | 59.0 |
| s.c. | 122.0 | 128.0 |
| p.o. | 790 | 840 |
| | brown rat | |
| i.v. | 11.8 | 13.0 |
| i.p. | 70.0 | 75.0 |
| s.c. | 295.0 | 283.0 |
| p.o. | 1110 | 1030 |
| | rabbit | |
| i.v. | 2.92 | --- |
| i.p. | 46.5 | --- |
| | guinea pig | |
| i.v. | 7.8 | --- |
| s.c. | 73.2 | --- |

One month toxicity test performed on brown rats with p.o. doses of 0.1; 1; 10 mg/kg has not shown any influence on the behaviour of animals, and neither haematological, biochemical and morphological indices nor the animal weight have been considerably influenced, except for the two highest concentrations of administered doses with which the increase in weight has been in some cases lower than average.

Chronical six month toxicity test performed on dogs (beagle) of both sexes with doses of 2; 10; 30 mg/kg resulted in no extensive changes in the weight of the dogs. No toxic effect in haematological, biochemical and morphological indicies has been observed with the two lower doses, except for minor changes in the heart activity, namely by influencing the pulse conductivity and further by psychomotoric

unrest. With the two highest doses, a reduction in weight, disturbance in behaviour and mortality have occured. Within the dose range of up to 2 mg.kg of animal weight no proofs about any harmfulness of the substance in the six months period of p.o. administration in the entire group of tested dogs have been found.

During teratologic and embryotoxic experiments on mice with doses of 0.21; 2.1: 8.4: 21: 42; 84 mg kg of animal weight, which corresponds to 0.025; 0.25; 1; 2.5; 5; 10% $LD_{50}$ no changes in the weight of fetal implants, weights of the placenta and the amount of amniotic fluid have been proved. The occurrence of skeletal abnormalties in the experimental group was the same as in the control group to which no medicament was administered. Only with the highest dose of 84 mg.kg cleft palate in less than 10 per cent of the fetus was observed.

With the substance according to the above formula, in doses ranging from 2.5 x $10^{-5}$ to 2.5 x $10^{-3}$ M/disc, no mutagenic effects in experiments in the detection of recessive lethal mutations with Drosophila melanogaster have been found. Further neither qulitative nor quantitative mutations in tests on Salmonella typhimurium and Escherichia coli as well as mutation changes in reparative tests effected on the same species have been observed.

The antidiarrhoeal medicament according to the above formula, ie different salts of (±)-trans-2(1-pyrrolidinyl)cyclohexyl ester of 3-(n)-pentyloxycarbanilic acid, can be prepared by dissolving the substance in water or aqueous solutions, or other liquids, semi-liquid bases, or possibly, in suitable solvents such as, for liquid forms, usual stabilizing admixtures (eg phosphate buffer), emulsifiers (sorbimacrogels), suspension or emulsion stabilizers (eg esters of cellulose, colloidal oxide of silicon, bentonite), and for solid form, by adding usual auxiliary substances (eg. starch, lactose, methylcellulose, gelatine, dextrane, magnesium strearate, or microcrystalline cellulose). The presupposed and low therapeutical dose of the medicament does not influence the base medicament form, its decomposition, disintegration or the like.

The compound according to the formula, ie the active substance, is prepared according to the mentioned Czechoslovak patents, Nos. 125,666 and 126,102.

EXAMPLE 1 - Antidiarrhoeal drug in capsule form

Capsules (22 mm length, 8 mm diameter) were filled with a mixture of 20 g of the substance and 60 g of lactose. One capsule contained 20 mg of the active substance.

Similarly there were prepared capsules with 10 mg doses of active substance in that an amount of the substance corresponding to said 10 mg does was added to lactose, and the capsules were filled with the mixture. Thus one capsule contained 10 mg of the active substance.

EXAMPLE 2 - Antidiarrhoeal drug in tablet form

10 g of active substance was mixed with 60 g of lactose and 138g of starch, whereupon the mixture was wetted with the necessary amount of starch hydrogel. The mixture was granulated and homogenized and 2 grams of magnesium strearate were added thereto.

The mixture was then presssed to tablets of about 250 mg weight and 5 mm diameter. One tablet contained a 10 mg dose of the active substance.

EXAMPLE 3 - Antidiarrhoeal drug in suspension form

500 mg of the substance were dissolved in 20 ml of distilled water and homogenized together with a suspension prepared from 20 mg of colloidal oxide of silicon. The resulting suspension contained 10 mg of the active substance in 5 millilitres.

**Claims**

1. An antidiarrhoeal therpeutic agent, characterized in that it contains as the active component the base (±) - trans 2 (1 -pyrrolidinyl) cyclohexylester of 3-(n)-pentyloxycarbanilic acid of the formula

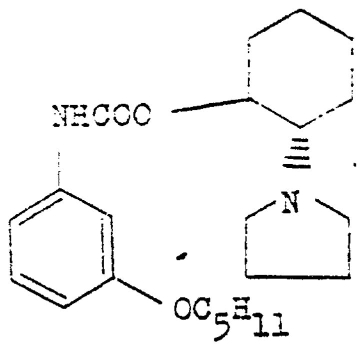

this base or its salts with pharmaceutically acceptable acids.

2. An agent according to Claim 1, wherein the salt is a hydrochloride.

3. An agent according to Claim 1 or 2, wherein the active component is mixed with a physiologically harmless vehicle.

4. An antidiarrhoeal therapeutic agent in the form according to Example I, II or III.